# DEMANDE DE BREVET EUROPEEN

(11) **EP 3 871 760 A1**
(43) Date de publication de la demande: **01.09.2021**
(21) Numéro de dépôt: 21159954.3
(22) Date de dépôt: 01.03.2021
(51) Int. Cl.: B01F 5/00, B01F 5/02, B01F 5/10, B01F 3/04, B01F 13/02, C12M 1/00, C12M 1/09

(54) **SYSTÈME D AGITATION ET BASSIN MUNI D'UN TEL SYSTÈME D AGITATION**

(30) Priorité: 27.02.2020 FR 2001969
(71) Demandeur: Nénuphar, 66370 Pézilla la Rivière (FR)
(72) Inventeur: DUONG, Frédéric, 66370 PÉZILLA-LA-RIVIÈRE (FR)
(74) Mandataire: Delaveau, Sophie

(57) **Abrégé**

La présente invention propose un système d'agitation hydrodynamique qui génére un effet de circulation vortex centripète (1) d'une solution (20) contenue dans un bassin (2) délimité par un bord (2a) et présentant un centre (C).

Selon l'invention, le système d'agitation comprend :
• un moyen d'aspiration (11a) de la solution au centre et au fond du bassin ;
• un circuit de transport (12) de la solution aspirée depuis le centre du bassin vers une première buse d'éjection (14) ayant une direction d'éjection tangentielle au bord (2a) du bassin (2) et sensiblement parallèle à une surface libre (21) de la solution (20) de manière, en utilisation, à éjecter la solution tangentiellement par rapport au bord du bassin et parallèlement à la surface libre de la solution, et à générer un courant de circulation centripète en spiral autour du centre du bassin

## Description

La présente invention concerne un système d'agitation et un bassin muni d'un tel système d'agitation, notamment pour équiper des bassins photo-bioréacteurs de production de microalgues alimentaires ou utilisées pour le traitement biologique des eaux potables (nitrates) et également pour un postraitement en sortie de station d'épuration (couramment appelée « STEP »).

Le développement de la culture algale, en particulier microalgale en vue d'obtenir un biocarburant photosynthétique, des aliments humains ou animaux ou des composés nutraceutiques issus de la biomasse algale, ne pourra être assuré à grande échelle qu'en réduisant significativement le coût de construction des bassins de culture et de leur coût d'exploitation. En outre, la production algale n'est pas réservée aux seules régions chaudes et ensoleillées, et il faut envisager des solutions techniques permettant d'augmenter la production algale dans des pays situés aux latitudes des régions tempérées, tout en minimisant les besoins en chaleur nécessaire au maintien à la température de croissance adaptée selon les souches de microalgues.

On connaît déjà des bassins en circuit fermé, tel que celui décrit dans le document WO 2013/011448. Il comprend un bassin fermé par un support de manches en matériau souple remplies de liquide pour capter et transmettre de la lumière au travers de la solution algale contenue dans le bassin.

Si ce bassin a permis de résoudre de nombreux problèmes techniques et économiques tels que l'augmentation de la productivité du bassin et la réduction des coûts de fabrication des manches cylindriques qui diffusent la lumière, il nécessite encore un équipement relativement conséquent, une fabrication de composants coûteuse et complexe à mettre en œuvre. En outre, cette technologie fermée ne s'applique qu'à des petits bassins pour la production de spiruline, de sorte qu'elle ne permet pas une production de microalgues à grande échelle.

En outre, la concentration de lumière vers les manches est complexe, car elle fait appel à des lentilles mobiles qui doivent être dirigées dynamiquement et précisément vers les manches.

Par ailleurs, la surface d'exposition à la lumière est restreinte à la surface des orifices du support dans lesquels débouchent les manches, le reste du support occultant l'intégralité de la surface libre du bassin.

Cette technologie souffre également d'un problème d'encrassement externe des manches par le développement d'un biofilm au contact entre les manches et la solution algale. Il est donc nécessaire de vider régulièrement les manches puis de les retirer de la solution afin de les nettoyer.

Afin de résoudre les problèmes précédents, l'inventeur a développé un dispositif 1 de capture, de transport et de diffusion de lumière dans une solution contenue dans un réservoir qui a fait l'objet de la Demande de brevet FR1801155 et qui est illustré en figure 1.

Le dispositif 1 est plongé dans un bassin B contenant la solution algale S. Le dispositif comprend une manche cylindrique M transparente destinée à être remplie d'un liquide propre à transmettre et diffuser la lumière, et une cloche C étanche, transparente et formée par deux parois séparées par un interstice rempli de gaz. La cloche comprend une partie en dôme D prolongée par une partie tubulaire T, une première extrémité de la manche M étant destinée à être bloquée contre la cloche de sorte qu'en position d'utilisation, la cloche flotte librement dans la solution et maintient émergée au moins la partie en dôme D et tout en assurant la verticalité de la manche M. La cloche est remplie d'eau avec ou sans additifs.

Ces dispositifs, qui captent la lumière naturelle et la conduisent dans la solution algale, flottent librement à la surface du bassin, grâce à leur structure en cloche équipée d'une double paroi étanche.

Cependant, le flottage libre aléatoire sur toute la surface du bassin peut conduire à un agglutinement des dispositifs dans une partie du bassin, réduisant la production d'algue dans l'autre partie du bassin qui bénéficie, alors, d'une distribution d'éclairement moindre. C'est particulièrement vrai en cas de vent.

Ce problème d'agglutinement se pose également pour tout autre dispositif flottant librement à la surface d'un bassin. On pense, par exemple, à des systèmes de mesure ou des systèmes de nutrition des algues ou de poissons, dont le mouvement doit être le plus aléatoire possible et couvrir la surface du bassin de manière uniforme (au moins d'un point de vue statistique) pour effectuer des mesures pertinentes ou assurer une nutrition homogène.

Pour résoudre le problème d'agglutinement des dispositifs de capture, de transport et de diffusion de lumière, les Demandes de brevet FR1902221 et FR1902222 au nom de la déposante décrivent des photo-bioréacteurs comprenant des carrousels d'agitation de la solution qui utilisent le fait que des dispositifs flottants indépendants et libres soient présents à la surface du bassin, et qui déplacent directement les dispositifs flottants et non la solution elle-même pour ne pas abîmer les algues qui sont souvent fragiles et ne supporte pas les contraintes mécaniques.

Quel que soit le système proposé, l'objectif est de tendre vers une homogénéité parfaite de la solution, c'est-à-dire que la concentration d'algues est identique, quel que soit l'endroit du bassin et la densité des microalgues.

Cependant, en pratique, la Demanderesse s'est aperçue que les solutions précédentes souffrent de certains défauts qui peuvent être rédhibitoires si l'on souhaite augmenter la taille des bassins. En effet, ces systèmes sont coûteux, car ils nécessitent un motoréducteur très puissant pour permettre la rotation des carrousels. En outre, si l'on constate une bonne homogénéité transversale, on constate également une mauvaise homogénéité verticale (c'est-à-dire en profondeur). Il y a ainsi une stratification des algues qui aboutit à la mort d'une grande partie d'entre elles qui se retrouve au fond du bassin. Ce phénomène est d'autant plus présent que le bassin est profond.

Or, il est nécessaire de pouvoir proposer un système d'agitation permettant la culture d'algues dans des bassins de grandes dimensions, à la fois latérales et verticales.

Il est également nécessaire d'assurer un brassage de la solution algale sans soumettre les algues à des contraintes mécaniques, c'est-à-dire sans les soumettre à un brassage mécanique trop intense.

Il est également souhaitable d'améliorer l'efficacité de la récolte, c'est-à-dire la quantité d'algues récupérée par unité de volume extrait.

Bien entendu, ces problèmes se posent également dans les bassins de STEP, même si c'est pour des raisons différentes. En effet, dans ces bassins remplis d'une solution d'eau et de particules (matière organique, graisses, sable, etc.) on ne cherche pas à augmenter la biomasse des micro-organismes, mais à favoriser le brassage pour que ces derniers soient en contact avec la matière organique pour la dégrader. En outre, on cherche également à extraire certaines particules indésirables, c'est-à-dire récupérer les particules solides en suspension, telles que le sable, pour l'éliminer. Un système d'agitation permettant de faciliter la récupération des particules en suspension et de favoriser le brassage est donc également nécessaire dans ce domaine.

Dans la suite de la présente description, on parlera indifféremment d'algues ou de particules.

L'idée qui sous-tend l'invention va totalement à l'encontre des prérequis de l'art antérieur qui tendent à maximiser l'homogénéité de la solution complète, c'est-à-dire transversalement et verticalement, par un brassage mécanique. En effet, l'invention propose créer les conditions d'une hétérogénéité transversale, c'est-à-dire entre le centre et le bord du bassin, et à corriger cette hétérogénéité transversale par un flux hydrodynamique sans mouvement mécanique de cisaillement pour permettre un meilleur mélange de la solution, aboutissant à une bonne homogénéité globale et une augmentation très significative de la production d'algue, en réduisant le taux de mortalité des algues. Cela permet concomitamment d'améliorer le rendement de récolte puisque cette dernière se fait à l'endroit où les algues (ou les particules) sont déplacées par le système selon l'invention. Enfin, les moyens mis en œuvre étant hydrodynamiques, ils sont respectueux de l'intégrité des algues, ce qui permet de cultiver des espèces d'algues fragiles.

L'invention est avantageusement mise en œuvre avec des dispositifs de capture de lumière conformes à ceux décrits dans la demande de brevet FR1801155 à la surface de la solution algale, ce qui augmente significativement la productivité algale.

À cette fin, l'invention a pour objet un système d'agitation d'une solution contenue dans un bassin en utilisation, le bassin étant délimité par un bord et présentant un centre, le système d'agitation comprenant :
- un moyen d'aspiration de la solution situé au centre et à proximité du fond du bassin ;
- un circuit de transport de la solution aspirée depuis le centre du bassin vers une première buse d'éjection ayant une direction d'éjection tangentielle au bord du bassin et sensiblement parallèle à une surface libre de la solution de manière, en utilisation, à éjecter la solution tangentiellement par rapport au bord du bassin et parallèlement à la surface libre de la solution, et à générer un courant de circulation centripète en spiral autour du centre du bassin.

Selon l'invention, la solution est avantageusement une solution algale ou une solution contenant des microalgues.

La portée de l'invention s'étend ainsi également à un bassin rempli d'une solution algale, qui comprend un système d'agitation comprenant :
- un moyen d'aspiration de la solution algale positionné au centre et au fond du bassin ;
- un circuit de transport de la solution algale aspirée depuis le centre du bassin vers une première buse d'éjection ayant une direction d'éjection tangentielle au bord du bassin et sensiblement parallèle à une surface libre de la solution de manière, en utilisation, à éjecter la solution tangentiellement par rapport au bord du bassin et parallèlement à la surface libre de la solution algale, et à générer un courant de circulation centripète en spiral autour du centre du bassin.

Selon d'autres modes de réalisation, qui peuvent être combinés entre eux et qui se rapportent au système d'agitation et au bassin précédemment définis:
- le système d'agitation peut comprendre au moins une deuxième buse d'éjection ayant une direction d'éjection complémentaire à la direction d'éjection de la première buse d'éjection pour renforcer le courant de circulation centripète en spiral généré par la première buse ;
- le circuit de transport peut comprendre une dérivation agencée entre le moyen d'aspiration et la ou les buses d'éjection, la dérivation étant munie d'une vanne de dérivation et aboutissant à un bac de collecte de la solution ;
- la dérivation peut comprendre un filtre ;
- le moyen d'aspiration peut comprendre une pompe reliée à un tube d'aspiration ;
- le moyen d'aspiration peut comprendre une buse d'injection de bulles d'air comprimé avec ou sans ajout de CO2 débouchant dans un tube d'aspiration ;
- le système d'agitation peut comprendre, en outre, un manchon de récolte ;
- avantageusement, le manchon de réculte est agencé de manière coaxiale avec le tube d'aspiration et monté coulissant verticalement entre une position de culture dans laquelle manchon de récolte est agencé hors de la solution, et une position de récolte dans laquelle le manchon de récolte est agencé au fond du bassin, le manchon de récolte et le tube d'aspiration étant séparé par un espace annulaire de largeur déterminée ;
- le circuit de transport peut être monté mobile en hauteur pour permettre un réglage en hauteur de la position de la ou des buses d'éjection en fonction du niveau de la surface libre de la solution dans le bassin ;
- la ou chaque buse d'éjection peut être munie d'un déflecteur agencé au moins au niveau de la surface libre de la solution, de sorte que la ou chaque buse d'éjection est placée entre le bord du bassin et le déflecteur ;
- le déflecteur peut être monté flottant pour pouvoir être toujours à la surface libre de la solution, quel que soit le niveau de la surface libre de la solution dans le bassin ;
- le circuit de transport peut comprendre un tube central d'aspiration relié à un bras radial tubulaire agencé radialement entre le tube central d'aspiration et le bord du bassin, le bras radial tubulaire étant relié à un tube d'injection ayant une extrémité munie de ladite première buse d'éjection orientée de manière à éjecter la solution tangentiellement par rapport au bord du bassin et à la surface libre de la solution, et à générer un courant de circulation centripète en spiral autour du centre du bassin ;
- le circuit de transport peut comprendre au moins un deuxième bras radial tubulaire agencé radialement entre le tube central d'aspiration et le bord du bassin, ledit au moins un deuxième bras radial tubulaire étant relié à un tube d'injection ayant une extrémité munie de ladite au moins une deuxième buse d'éjection ; et/ou
- la ou les buses d'éjection peuvent être agencées dans la solution.

L'invention permet ainsi d'adresser simultanément tous les problèmes précédents, notamment celui de la productivité et de la baisse du taux de mortalité, tout en permettant une récolte des algues facilitée, ainsi qu'une agitation homogène et respectueuse de la solution algale.

L'invention a également pour objet un bassin pour la production d'une solution algale comprenant au moins un système d'agitation précédent.

Selon d'autres modes de réalisation, qui peuvent être combinés entre eux :
- le bassin peut comprendre une pluralité de dispositifs en flottage libre à la surface de la solution ; et/ou
- les dispositifs en flottage libre peuvent être des dispositifs de capture, de transport et de diffusion de lumière comprenant une manche cylindrique, en matériau transparent à la lumière transportée et diffusée, la manche étant destinée à être remplie d'un liquide propre à transmettre et diffuser la lumière et à équilibrer la pression extérieure pour maintenir la manche sous forme cylindrique, une cloche étanche, transparente à la lumière captée, transportée et diffusée, , la cloche comprenant une partie en dôme prolongée par une partie tubulaire équipée d'un flotteur annulaire, une première extrémité de la manche étant destinée à être bloquée contre la cloche de sorte qu'en position d'utilisation, la cloche flotte librement dans la solution et maintient au moins la partie en dôme émergée et la manche remplie de liquide immergée et étendue verticalement sous la cloche en adoptant une forme cylindrique.

D'autres caractéristiques de l'invention seront énoncées dans la description détaillée ci-après, faite en référence aux dessins annexés, qui représentent, respectivement :
[Fig. 1], une vue schématique en coupe d'un dispositif de capture, de transport et de diffusion de lumière du document FR1801155 en position d'utilisation dans un réservoir ;
[Fig. 2], une vue schématique en coupe d'un premier exemple de réalisation d'un système d'agitation selon l'invention en position d'utilisation dans un bassin ;
[Fig. 3], une vue schématique en coupe d'un deuxième exemple de réalisation d'un système d'agitation selon l'invention en position d'utilisation dans un bassin ;
[Fig. 4], une vue schématique de dessus du mode de réalisation de la figure 2 illustrant le courant spiral centripète ;
[Fig. 5], une vue schématique de dessus d'un agrandissement de la figure 4 et illustrant un déflecteur mécanique évitant l'agglutinement de dispositifs flottant à la surface de la solution, tels que les dispositifs de capture, de transport et de diffusion de lumière du document FR1801155 ;
[Fig. 6], une vue schématique en coupe d'un deuxième mode de réalisation du moyen d'aspiration mettant en œuvre de l'air comprimé ;
[Fig. 7], une vue schématique en coupe d'un bassin équipé d'un système d'agitation selon l'invention combiné à un manchon de récolte en position de culture ;
[Fig. 8], une vue schématique en coupe du bassin de la figure 7 dans lequel le manchon de récolte est en position de récolte ;
[Fig. 9], une vue schématique en coupe d'un bassin équipé d'un système d'agitation classique combiné à un manchon de récolte en position de culture ;
[Fig. 10], une vue schématique en coupe du bassin de la figure 9 dans lequel le manchon de récolte est en position de récolte ; et
[Fig. 11], une vue schématique en coupe d'un dispositif de capture, de transport et de diffusion de lumière selon une seconde alternative.

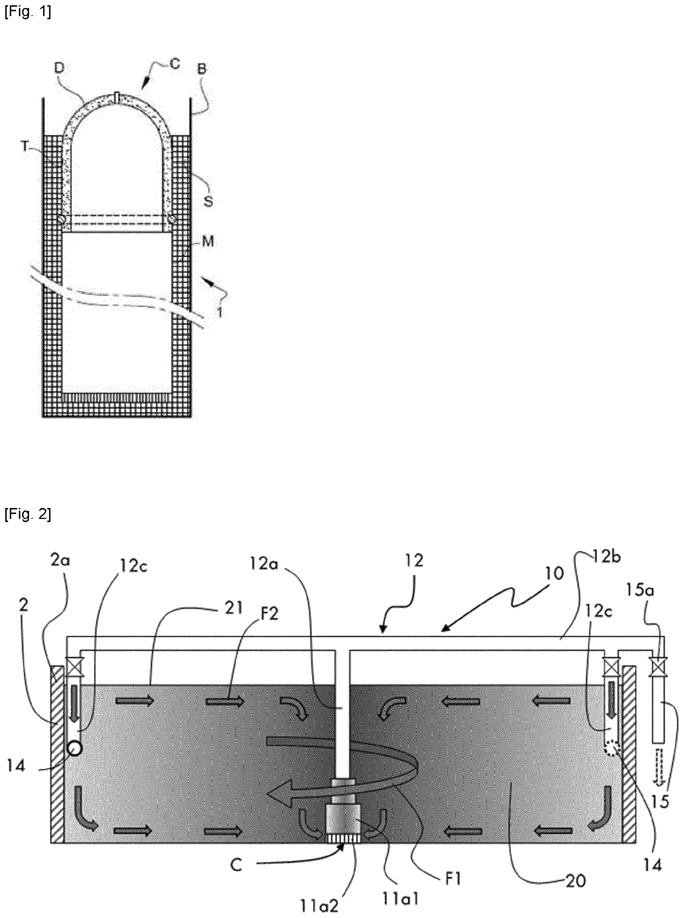

La figure 2 l'illustre un bassin 2, muni d'un système d'agitation 10, selon l'invention, vu en coupe. Le bassin est délimité par un bord 2a et présente un centre C.

Le bassin est rempli d'une solution 20 présentant une surface libre 21. La solution comprend un liquide et des particules en suspension, telle que des algues.

Le système d'agitation 10 selon l'invention comprend un moyen d'aspiration 11 agencée pour aspirer la solution contenue dans le bassin 2 au centre C du bassin. Dans le mode de réalisation illustré en figure 2, le moyen d'aspiration est une pompe immergée 11a1 qui est posée au fond (ou à proximité du fond) et au centre du bassin 2. La pompe 11a1 comprend une première extrémité d'aspiration munie d'une grille 11a2 agencée au fond (ou à proximité du fond) et au centre du bassin 2, et une deuxième extrémité reliée à un tube central d'aspiration 12a d'un circuit de transport 12 de la solution aspirée depuis le fond et le centre C du bassin, par le moyen d'aspiration 11 vers une première buse d'éjection 14.

Dans le mode de réalisation illustrée, le circuit de transport 12 comprend une deuxième buse d'éjection 14. Bien entendu, le système d'agitation selon l'invention peut comprendre d'autres buses d'éjection 14 identiques à la première et à la deuxième.

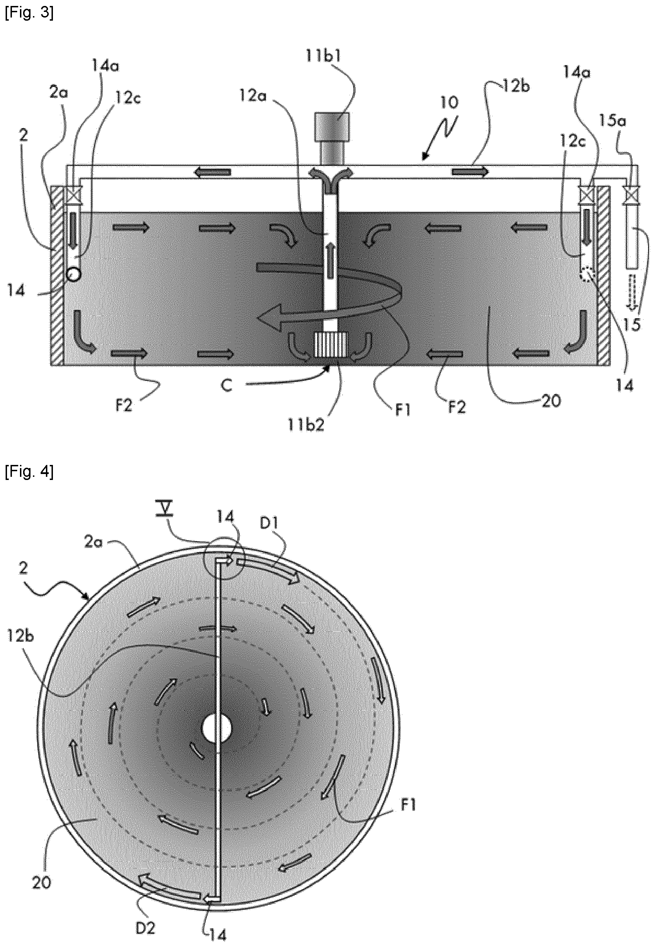

Selon l'invention, la première buse d'éjection 14 est agencée de manière à ce que sa direction d'éjection D1 (voir figure 4) soit tangentielle au bord 2a du bassin 2 et sensiblement parallèle à la surface libre 21 de la solution 20. Agencée de cette manière, la première buse d'éjection 14 éjecte au niveau du bord 2a du bassin la solution provenant du centre du bassin, tangentiellement par rapport au bord 2a de du bassin et parallèlement à la surface libre 21 de la solution, ce qui génère un courant de circulation centripète F1, en spirale autour du centre C du bassin. Ce courant est appelé, par la suite, courant vortex. Ce courant vortex déplace les particules ou les algues vers le centre du bassin. Si la pompe ne fonctionnait pas les particules auraient tendance à se concentrer au centre du bassin, créant un gradient de concentration croissant depuis le bord du bassin vers le centre, c'est-à-dire une hétérogénéité transversale. Grâce au pompage, cette hétérogénéité est corrigée et évitée car les particules qui sont déplacées par le courant vortex vers le centre du bassin sont réinjectées tangentiellement au bord du bassin et tendent à revenir vers le centre. Finalement, la solution est globalement homogène.

Le mode de réalisation illustré comprenant une deuxième buse d'éjection 14. Celle-ci est agencée de manière à ce que sa direction d'éjection D2 (voir figure 4) soit complémentaire à la direction d'éjection D1 de la première buse d'éjection 14 pour renforcer le courant vortex F1 généré par la première buse 14. En d'autres termes, la deuxième buse d'éjection 14 (ou les autres buses d'éjection 14) doit être agencée de manière à ce que les courants générés par chaque buse d'éjection s'ajoutent et ne se contrarient pas et contribuent ensemble à la génération d'un seul courant vortex général F1.

La figure 3 illustre un mode de réalisation alternatif dans lequel le moyen d'aspiration 11 comprend une pompe externe 11b1 agencée à l'extérieur de la solution. Cette pompe 11b1 est connectée à un tube d'aspiration 12a comprenant une extrémité d'aspiration munie d'un filtre 11b2, et agencée au centre et au fond (ou à proximité du fond) du bassin de manière à aspirer la solution située au centre et au fond du bassin.

Le système d'agitation selon l'invention est hydrodynamique, c'est-à-dire qu'il génère un courant d'agitation dans le bassin sans agitateur mécanique, et donc sans mouvement mécanique de cisaillement, uniquement par une injection de solution tangentiellement au bord du bassin.

Typiquement, pour un bassin de 10 m de diamètre, de 1 m de profondeur, on choisira des tubulures pour le un circuit de transport de 80 mm de diamètre, une pompe ayant un débit de 10 m3/h, et des buses d'éjection 14 de 50 mm de diamètre de sortie. On pourra obtenir le courant vortex selon l'invention même avec une faible vitesse d'éjection, mais plus on augmentera la vitesse d'injection des buses 14, plus on obtiendra un déplacement des algues (ou des particules) vers le centre du bassin rapidement.

Le circuit de transport étant fixe par rapport au bassin, l'éjection de solution par les buses génère un courant de circulation centripète en spirale F1 se dirigeant vers le centre du bassin, comme illustré en figure 14. Ce faisant, les particules contenues dans le bassin tendent à se regrouper et à se concentrer vers le centre du bassin dans le sens des flèches F2, générant potentiellement un gradient de concentration croissant depuis le bord du bassin vers le centre du bassin. Grâce au pompage, cette concentration est évitée tout en facilitant la collecte et en augmentant le rendement de collecte.

On constate également que le courant généré par les buses entraîne les particules sur toute la hauteur du bassin de sorte que le déplacement des particules est relativement homogène verticalement, c'est-à-dire entre le fond et la surface de la solution, contrairement à ce qui est obtenu dans l'art antérieur.

Avantageusement, les buses 14 sont agencées dans la solution, et non au-dessus de la solution 20. De préférence, les buses sont agencées dans la première moitié de la solution, c'est-à-dire entre la surface libre 21 et la mi-profondeur du bassin. Avantageusement, le système d'agitation selon l'invention comprend un moyen de réglage en hauteur des buses afin d'obtenir un courant vortex et sur toute la hauteur de la solution le plus rapidement possible.

De préférence, comme illustré aux figures 2 et 3, le circuit de transport 12 comprend une dérivation 15 agencée entre le moyen d'aspiration 11 et la ou les buses d'éjection 14. Cette dérivation est munie d'une vanne de dérivation 15a et aboutit à un bac de collecte (non illustré) de la solution, dans le sens de la flèche F3. Avantageusement, la dérivation 15 comprend un filtre de sorte que la solution sortant de la dérivation soit filtrée avant son entrée dans le bac de collecte de la solution. Il peut s'agir, par exemple, d'un filtre à tamis pouvant être retirés et lavés pour une utilisation ultérieure.

Dans un mode de réalisation avantageux, le circuit de transport 12 de la solution comprend également des vannes d'équilibrage 14a permettant de régler le débit de la solution soit entre deux ou plusieurs buses d'éjection soit dans la dérivation de récolte.

Le circuit de transport peut être constitué de tubulures rigides, ou de tubulures souples soutenues par un châssis rigide.

Le circuit de transport 12 comprend généralement un tube central d'aspiration 12a relié d'une part au moyen d'aspiration 11 et d'autre part à un bras radial tubulaire 12b agencé radialement entre le tube d'aspiration central 12a et le bord 2a du bassin. Le bras radial tubulaire 12b est relié à un tube d'injection 12c ayant une extrémité munie d'une buse d'éjection 14 telle que décrite précédemment. Avantageusement, comme dans les exemples de réalisation illustrés, le circuit de transport 12 comprend au moins un deuxième bras radial tubulaire 12b également agencé radicalement entre le tube central d'aspiration 12a et le bord 2a du bassin. Ce deuxième bras radial tubulaire 12b est relié à un deuxième tube d'injection 12c ayant également une extrémité munie d'une buse d'éjection 14 telle que décrite précédemment. Les bras radiaux tubulaires 12b peuvent être symétriques ou non. Cependant, en étant symétriques, cela répartit les forces appliquées à la solution et on assure une bonne régularité du courant vortex F3.

Le circuit de transport de solution et les buses d'éjection 14 étant immobiles par rapport au bassin, il peut être nécessaire, lorsque le bassin est utilisé en combinaison avec des dispositifs 1 de capture, de transport et de diffusion de lumière du document FR 1801155 tel que celui représenté sur la figure 1, de s'assurer que ces dispositifs flottants ne restent pas bloqués contre la buse ou les conduites tubulaires du circuit de transport.

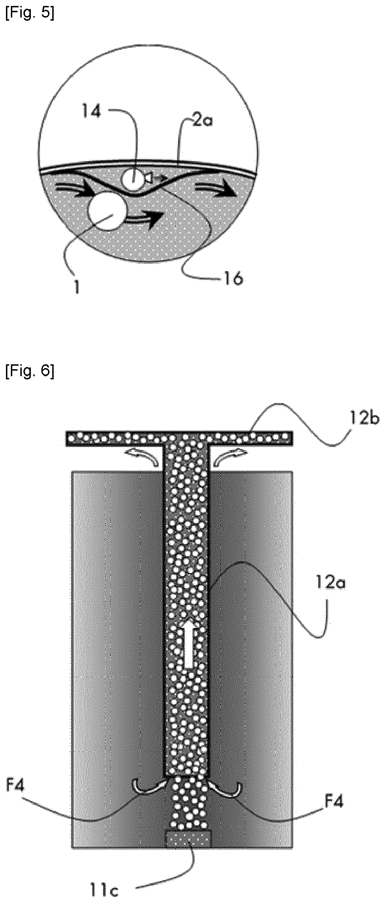

À cette fin, comme illustré en figure 5, l'invention prévoit que la ou chaque buse d'éjection 14 soit munie d'un déflecteur 16 agencé, au moins au niveau de la surface libre de la solution de sorte qu'en utilisation, la buse ou chaque buse 14 soit placée entre le bord 2a du bassin 2 et le déflecteur 16.

Plus précisément, la buse d'éjection 14 étant dans la solution, le déflecteur 16 est agencé au niveau de la surface libre de la solution de sorte qu'en utilisation, le tube d'injection 12c soit placé entre le bord 2a du bassin 2 et le déflecteur 16. Ce dernier protège donc le tube d'injection 12c et empêche les dispositifs 1 de se concer contre lui.

Avantageusement, le déflecteur est monté flottant pour pouvoir toujours être à la surface de la solution, quel que soit le niveau de la surface libre 21 de la solution 20 dans le bassin. Ainsi, lorsque ce niveau monte ou descend selon les conditions d'utilisation, le déflecteur est toujours en position d'utilisation et évite à tout dispositif 1 flottant à la surface de se bloquer entre le bord du bassin et la ou les buses d'éjection.

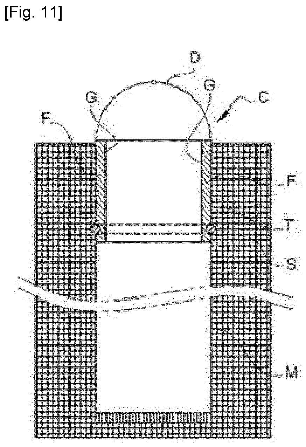

Alternativement au dispositif de capture, de transport et de diffusion de lumière de la figure 1, il peut être prévu un dispositif de capture, de transport et de diffusion de lumière tel que celui représenté sur la figure 11 qui est identique à celui de la figure 1 en ce qu'il comporte une manche cylindrique M transparente destinée à être remplie d'un liquide propre à transmettre et diffuser la lumière, et une cloche C étanche, transparente. La cloche comprend une partie en dôme D prolongée par une partie tubulaire T, une première extrémité de la manche M étant destinée à être bloquée contre la cloche de sorte qu'en position d'utilisation, la cloche flotte librement dans la solution et maintient émergée au moins la partie en dôme D et tout en assurant la verticalité de la manche M. Selon cette alternative, la cloche C présente une paroi simple. Par ailleurs, le dispositif comporte avantageusement un flotteur annulaire F ménagé au niveau de la paroi annulaire de la partie tubulaire T, et un guide d'onde réfléchissant G également annulaire qui est situé contre la face interne du flotteur annulaire F et qui permet d'optimiser la diffusion de la lumière. Comme pour le dispositif de la figure 1, la cloche est remplie d'eau avec ou sans additifs.

La figure 6 illustre un mode de réalisation alternatif du moyen d'aspiration 11. Dans ce mode de réalisation, le moyen d'aspiration comprend une buse 11c d'injection de bulles d'air comprimé, avec ou sans adjonction de dioxyde de carbone (CO2) pour favoriser la photosynthèse des algues et donc leur développement, la buse d'injection 11c étant agencée au centre et au fond du bassin, et en vis-à-vis d'un tube d'aspiration 12a.

En utilisation, la buse 11c génère un panache de bulles d'air comprimé qui s'élève dans le tube d'aspiration 12a en entraînant avec lui la solution située aux abords immédiats du tube d'aspiration 12a, comme illustré par les flèches F4.

Outre la fonction d'aspiration obtenue par ce dispositif, ce dernier permet d'augmenter l'apport de CO2 nécessaire à la photosynthèsede la solution tout en limitant les contraintes mécaniques sur les particules contenues dans la solution.

Le courant vortex généré par le système d'agitation selon l'invention, déplace les particules vers le centre du bassin, améliorant et facilitant la récolte de ces particules puisqu'il n'est plus nécessaire de déplacer la pompe de récolte dans tout le bassin.

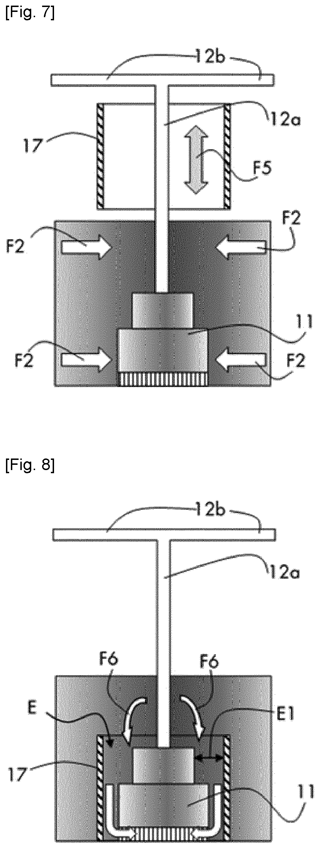

Il est possible d'améliorer et d'accélérer encore la récolte des particules au centre du bassin, en équipant le système d'agitation selon l'invention, avec un manchon de récolte 17, tel qu'illustré aux figures 7 et 8.

Comme illustré en figure 7, le manchon de récolte 17 est agencé de manière coaxiale avec le tube central d'aspiration 12a. Il est en outre monté coulissant verticalement, dans le sens de la flèche F5 entre une position de culture, dans lequel le manchon de récolte est agencé hors de la solution (figure 7), et une position de récolte dans lequel le manchon de récolte est agencé au fond du bassin (figure 8). Dans cette dernière position, le manchon et le tube d'aspiration sont séparés par un espace annulaire E de largeur E1 déterminée. Cette largeur E1 doit être suffisante pour permettre un débit d'aspiration compatible avec l'utilisation industrielle et pour préserver l'intégrité des algues, tout en étant suffisamment restreinte pour n'isoler que la partie la plus concentrée de la solution obtenue après une phase de concentration (voir ci-après).

En position de culture (figure 7), le manchon 17 est en dehors de la solution 20 et le courant vortex généré par le système d'agitation selon l'invention agite normalement la solution et tend à déplacer les particules vers le centre du bassin, dans le sens des flèches F2. Concommittament, la pompe aspire la solution au centre et au fond du bassin et la recycle vers les buses d'éjection 14.

Pour récolter les particules, on ferme temporairement les vannes 14a et on arrête la pompe. L'inertie du fluide et l'effet vortex tendent à concentrer les particules au centre du bassin, ce que l'on appelle la phase de concentration.

Lorsque la soloution est concentrée au centre du bassin, on abaisse le manchon de récolte 17 selon l'invention, on redémarre la pompe et on ouvre la vanne 15a pour que la solution soit pompée vers le bac de collecte.

Le manchon de récolte 17 isole du reste du bassin les particules concentrées au centre du bassin et l'aspiration générée par le moyen d'aspiration 11 est canalisée, de sorte que la colonne de particules agencées au centre du bassin est prioritairement aspirée très rapidement dans le sens des flèches F6. Bien entendu, de la solution située à l'extérieur du manchon est également aspirée.

Une fois la récolte effectuée, le manchon de récolte 17 est remonté et le courant vortex généré par le système d'agitation selon l'invention reprend et déplace, à nouveau, les algues vers le centre du bassin puis sont aspirées et remises en circulation au niveau des buses 14.

La récolte ayant été faite, la concentration générale de la solution en algues a diminué, ce qui permet aux algues restantes d'accéder plus facilement à la lumière et accélère leur reproduction.

Dans un mode de réalisation préféré, le système selon l'invention comprend un capteur permettant de détecter la baisse de concentration en particules dans la solution aspirée, le capteur étant relié aux vannes 14a et 15a pour fermer la vanne 15a et rouvrir les vannes 14a et gérer l'alimentation de la pompe. Concomitamment le manchon de récolte 17 est relevé en position de culture.

Indépendamment du système d'agitation selon l'invention décrit précédemment, le manchon de récolte 17 peut être utilisé dans tout bassin nécessitant d'améliorer le rendement de récolte, y compris les bassins de l'art antérieur dans lesquelles il existe une stratification horizontale des particules, c'est-à-dire dans lesquels il y a une hétérogénéité verticale. En effet, certaines particules telles que les algues vivantes ou les graisses ont naturellement tendance à se concentrer vers le haut du bassin

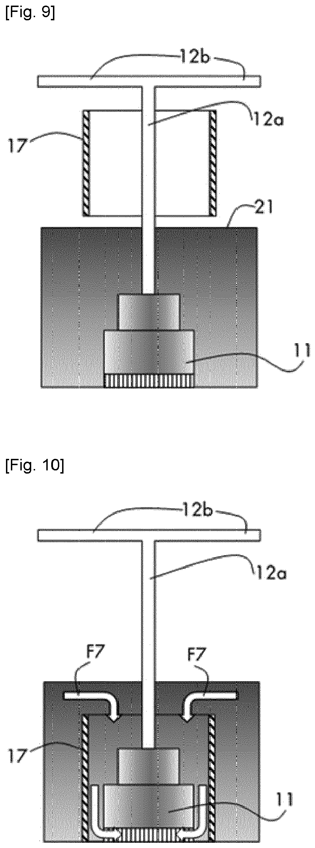

Cet exemple d'utilisation du manchon de récolte selon l'invention est illustré aux figures 9 (position de culture) et 10 (position de récolte).

En figure 9, la solution contenue dans le bassin, dépourvu du système d'agitation selon l'invention décrit précédemment, présente un gradient de concentration augmentant depuis le fond du bassin vers la surface libre 21 de la solution.

Le manchon de récolte 17 selon l'invention est alors abaissé, comme illustré en figure 10. La pompe de récolte 11 plongée dans le fond du bassin (au centre du bassin ou non) aspire la solution contenue dans le manchon de récolte 17. Cette aspiration attire alors la solution située au-dessus du manchon de récolte dans le sens des flèches F7, cette solution étant très concentrée en algues. On améliore ainsi, le rendement de récolte en aspirant prioritairement la solution la plus proche de la surface libre de la solution.

Avant la période de récolte, un arrêt de quelques minutes de l'agitation peut permettre de favoriser la stratification densimétrique naturelle et améliorer encore le rendement de récolte.

Grâce au système d'agitation selon l'invention, la photo période d'exposition aux rayons qui impacte directement la productivité est nettement augmentée. La photo inhibition en surface du bassin est réduite pendant les périodes intenses d'ensoleillement et le dispositif permet également d'assurer la récolte semi-continue avec l'ouverture d'une seule vanne.

Le système selon l'invention peut équiper des bassins existants de type « raceway » afin d'augmenter significativement leur productivité. Des bassins ouverts de grosse capacité et de plus grande profondeur permettront d'optimiser la production spécifique (objectif de productivité supérieur à 100 t/ha.an de matière sèche) et de réduire le coût global foncier, d'équipement et d'installation.

Le système selon l'invention peut également équiper les bassins de traitement biologique des eaux potables (nitrates) et en sortie de station d'épuration. Cela permet d'améliorer le brassage des microorganismes et favorise donc la dégradation de la matière organique.

En outre, comme pour les bassins de culture d'algues, en concentrant les déchets au centre du bassin, leur évacuation est facilitée. Elle est donc plus efficace en termes de rendement de récolte et plus efficace énergétiquement puisqu'à quantité de matière récoltée égale avec une STEP classique, la récolte prend moins de temps.

## Revendications

1. Système d'agitation (10) d'une solution (20) contenue dans un bassin (2) en utilisation, le bassin étant délimité par un bord (2a) et présentant un centre (C), **caractérisé en ce qu'**il comprend :
• un moyen d'aspiration (11, 11a, 11b, 11c) de la solution positionné au centre et au fond du bassin ;
• un circuit de transport (12) de la solution aspirée depuis le centre du bassin vers une première buse d'éjection (14) ayant une direction d'éjection (D1) tangentielle au bord (2a) du bassin (2) et sensiblement parallèle à une surface libre (21) de la solution (20) de manière, en utilisation, à éjecter la solution tangentiellement par rapport au bord du bassin et parallèlement à la surface libre de la solution, et à générer un courant de circulation centripète en spiral (F1) autour du centre du bassin.

2. Système d'agitation (10) selon la revendication 1, comprenant au moins une deuxième buse d'éjection (14) ayant une direction d'éjection (D2) complémentaire à la direction d'éjection (D1) de la première buse d'éjection pour renforcer le courant de circulation centripète en spiral (F1) généré par la première buse.

3. Système d'agitation (10) selon l'une quelconque des revendications 1 ou 2, dans lequel le circuit de transport (12) comprend une dérivation (15) agencée entre le moyen d'aspiration (11, 11a, 11b, 11c) et la ou les buses d'éjection (14), la dérivation étant munie d'une vanne de dérivation (15a) et aboutissant à un bac de collecte de la solution.

4. Système d'agitation (10) selon la revendication 3, dans lequel la dérivation (15) comprend un filtre.

5. Système d'agitation (10) selon l'une quelconque des revendications 1 à 4, dans lequel le moyen d'aspiration comprend une pompe (11a, 11b) reliée à un tube d'aspiration (12a).

6. Système d'agitation (10) selon l'une quelconque des revendications 1 à 4, dans lequel le moyen d'aspiration comprend une buse (11c) d'injection de bulles d'air comprimé avec ou sans adjonction de CO2 débouchant dans un tube d'aspiration (12a).

7. Système d'agitation (10) selon l'une quelconque des revendications 5 ou 6, comprenant, en outre, un manchon de récolte (17) agencé de manière coaxiale avec le tube d'aspiration (12a) et monté coulissant verticalement entre une position de culture dans laquelle manchon de récolte (17) est agencé hors de la solution, et une position de récolte dans laquelle le manchon de récolte est agencé au fond du bassin (2), le manchon de récolte (17) et le tube d'aspiration (12a) étant séparé par un espace annulaire (E) de largeur déterminée (E1).

8. Système d'agitation (10) selon l'une quelconque des revendications 1 à 7, dans lequel le circuit de transport (12) est monté mobile en hauteur pour permettre un réglage en hauteur de la position de la ou des buses d'éjection (14) en fonction du niveau de la surface libre (21) de la solution (20) dans le bassin (2).

9. Système d'agitation (10) selon l'une quelconque des revendications 1 à 8, dans lequel la ou chaque buse d'éjection (14) est munie d'un déflecteur (16) agencé au moins au niveau de la surface libre (21) de la solution (20), de sorte que la ou chaque buse d'éjection (14) est placée entre le bord (2a) du bassin et le déflecteur (16).

10. Système d'agitation (10) selon la revendication 9, dans lequel le déflecteur (16) est monté flottant pour pouvoir être toujours à la surface libre de la solution, quel que soit le niveau de la surface libre de la solution dans le bassin.

11. Système d'agitation (10) selon l'une quelconque des revendications 1 à 10, dans lequel le circuit de transport (12) comprend un tube central d'aspiration (12a) relié à un bras radial tubulaire (12b) agencé radialement entre le tube central d'aspiration (12a) et le bord (2a) du bassin, le bras radial tubulaire (12b) étant relié à un tube d'injection (12c) ayant une extrémité munie de ladite première buse d'éjection (14) orientée de manière à éjecter la solution tangentiellement par rapport au bord du bassin et à la surface libre de la solution, et à générer un courant de circulation centripète en spiral autour du centre du bassin.

12. Système d'agitation (10) selon la revendication 11 lorsqu'elle dépend de la revendication 2, dans lequel le circuit de transport (12) comprend au moins un deuxième bras radial tubulaire (12b) agencé radialement entre le tube central d'aspiration (12a) et le bord (2a) du bassin, ledit au moins un deuxième bras radial tubulaire (12b) étant relié à un tube d'injection (12c) ayant une extrémité munie de ladite au moins une deuxième buse d'éjection (14).

13. Système d'agitation (10) selon l'une quelconque des revendications 1 à 12, dans lequel la ou les buses d'éjection (14) sont agencées dans la solution.

14. Bassin (2) pour la production d'une solution algale (20), **caractérisé en ce qu'**il est rempli d'une solution algale et **en ce qu'**il comprend au moins un système (10) d'agitation selon l'une quelconque des revendications 1 à 13.

15. Bassin (2) selon la revendication 14, comprenant une pluralité de dispositifs (1) en flottage libre à la surface de la solution (20).

16. Bassin (2) selon la revendication 15, dans lequel les dispositifs (1) en flottage libre sont des dispositifs de capture, de transport et de diffusion de lumière comprenant une manche cylindrique (M), en matériau transparent à la lumière transportée et diffusée, la manche (M) étant destinée à être remplie d'un liquide propre à transmettre et diffuser la lumière et à équilibrer la pression extérieure pour maintenir la manche sous forme cylindrique, une cloche (D-T) étanche, transparente à la lumière captée, transportée et diffusée, , la cloche (D-T) comprenant une partie en dôme (D) prolongée par une partie tubulaire (T) équipée d'un flotteur annulaire, une première extrémité de la manche (M) étant destinée à être bloquée contre la cloche (D-T) de sorte qu'en position d'utilisation, la cloche flotte librement dans la solution et maintient au moins la partie en dôme (D) émergée et la manche (M) remplie de liquide immergée et étendue verticalement sous la cloche en adoptant une forme cylindrique.
